# EUROPEAN PATENT APPLICATION

(11) **EP 2 361 616 A1**
(43) Date of publication of application: **31.08.2011**
(21) Application number: 10197080.4
(22) Date of filing: 27.12.2010
(51) Int. Cl.: A61K 9/28

(54) **Extended release compositions for high solubility, high permeability active pharmaceutical ingredients**

(30) Priority: 25.12.2009 US 290178 P
(71) Applicant: Dexcel Pharma Technologies Ltd., 30600 Or Akiva (IL)
(72) Inventor: Onn, Liat, 14419, Tiberias (IL); Avramoff, Avi, 34403, Haifa (IL)
(74) Representative: Jones, Keith William

(57) **Abstract**

A novel composition comprising a tablet, comprising a highly soluble active pharmaceutical ingredient, which is then coated with a coating. The core is preferably a tablet. The coating preferably comprises any type of suitable extended release polymer, with the proviso that the polymer does not comprise an enteric polymer.

## Description

### FIELD OF THE INVENTION

The present invention relates to a novel formulation for a Biopharmaceutics Classification System (BCS) Class I drug, such as a metoprolol salt or tolterodine and methods of preparation and administration thereof, and in particular, to an extended release formulation comprising a coated tablet comprising such a BCS Class I drug.

### BACKGROUND OF THE INVENTION

The Biopharmaceutics Classification System (BCS) is a guide for predicting the intestinal drug absorption provided by the U.S. Food and Drug Administration, using the parameters of solubility and intestinal permeability. The solubility classification is based on a United States Pharmacopoeia (USP) aperture. The intestinal permeability classification is based on a comparison to the intravenous injection.

BCS Class I drugs have high permeability and high solubility. A drug is considered highly soluble when the highest dose strength is soluble in 250ml or less of aqueous media over the ph range of 1 to 7.5. A drug substance is considered highly permeable when the extent of absorption in humans is determined to be 90 % or more of the administered dose based on a mass-balance determination or in comparison to and intravenous dose.

Examples of BCS Class I drugs include metoprolol and tolterodine.

Metoprolol is a a selective Beta-1 blocker, used for treating various cardiovascular diseases such as hypertension. The active substance is generally provided in the form of a salt of metoprolol, such as metoprolol succinate or metoprolol tartrate.

Metoprolol succinate is described and claimed in US Patent No. 5081154.

US 6,827,947 to AstraZeneca relates to a film coating composition suitable for use in coating pharmaceutical formulations to provide modified release comprising a dispersion which includes: a) an acrylic polymer, b) a vinyl acetate polymer, and c) a water-containing liquid. The film coat is useful for the achievement of modified release from pharmaceutical formulations such as tablets, pellets, and so forth, including for metoprolol. However, the formulations are described as featuring pellets containing metoprolol which are coated with the above coating and which are only then compressed into tablets.

US 4,927,640 to Aktiebolaget Hassle teaches the use of very small tablets, for example using glass, onto which metoprolol is sprayed. The very small cores are then further coated with a coating; compression is not taught or suggested.

US 2007212416 to Glatt Air Techniques describes a composition which comprises uncoated pellets containing a pharmaceutical compound, such as metoprolol, which are dispersed in a matrix which comprises said pellets and a swellable polymer which is compressed into a tablet. The tablet is not coated.

US 2004185097 to Glenmark describes a formulation featuring multiple release modifying agents, such as for example polyethylene oxide. Single tablets are directly formed and are then coated.

US 20020132002 and US 20070264332 to Kos describe single matrix tablets, in which metoprolol or another active ingredient is embedded, which may then be coated.

EP 293347B1 to Aktiebolaget Hassle teaches a tablet containing metoprolol which is coated with an acrylic polymer coating.

EP 271438B1 to Ciba-Geigy teaches an oral osmotic system for the administration of metoprolol, comprising: a) an outer casing made of a semi-permeable material that is permeable to water and impermeable to the components of the core containing active ingredient; b) a core that comprises a mixture, which is sufficiently water-soluble to produce osmotic pressure, of 7.5 to 15 percent by weight poly-N-vinylpyrrolidone, about 5 percent by weight of a glidant or lubricant customary in the preparation of solid dosage forms, and 80 to 92.5 percent by weight of a moderately water-soluble, pharmaceutically acceptable metoprolol salt; and c) a passageway through the casing a) for transporting the constituents contained in the core into the surrounding aqueous body fluid.

WO 04/069234 to IPCA teaches a pharmaceutical composition comprising a matrix material having metoprolol, or a pharmaceutically acceptable salt thereof, dispersed therein, which is coated with a coating.

WO 08/014175 to Dr. Reddy teaches pellets which are first coated and then compressed to form tablets.

WO 08/012346 to Farmaprojects teaches granules which are first coated and then compressed to form tablets.

WO 07/073894 to Add Advanced Drug Delivery Technologies Ltd. relates to a pharmaceutical pellet composed of a spherical nucleus with a smooth surface containing the active substance and a coating of the nucleus, amounting to 40 - 60 w/w % of the nucleus, which controls the release of the active substance irrespective of pH, whereby the nucleus is composed of a starter nucleus not containing active substance and a layer of active substance positioned over it, whereby the starter nucleus includes one or more hydrocarbons, whereby the active substance is a metoprolol salt, particularly metoprolol succinate and whereby the coating includes polyvinyl acetate. The recitation of a coating which controls the release of the active substance regardless of pH effectively eliminates enteric coating polymers.

WO 07/052299 to Rubicon relates to a burst release dosage form.

WO 00/06129 to Lipha relates to pellets having a matrix for containing metoprolol, which are then coated.

Tolterodine, (R)-N,N-diisopropyl-3-(2-hydroxy-5-methylphenyl)-3-phenylpropanamine) is a muscarinic receptor antagonist that is used to treat or prevent overactive bladder and other problems related to urinary incontinence.

The major, active metabolite of tolterodine is (R)-N,N-diisopropyl-3-(2-hydroxy-5-hydroxymethylphenyl)-3-phenylpropanamine; the corresponding (S)-enantiomer to tolterodine is (S)-N,N-diisopropyl-3-(2-hydroxy-5-methylphenyl)-3-phenylpropanamine; the 5-hydroxymethyl metabolite of the (S)-enantiomer is (S)-N,N-diisopropyl-3-(2-hydroxy-5-hydroxymethylphenyl)-3-phenylpropanamine; and the corresponding racemate to tolterodine is (R,S)-N,N-diisopropyl-3-(2-hydroxy-5-methylphenyl)-3-phenylpropanamine.

Toleterodine, its corresponding (S)-enantiomer and racemate, and the preparation thereof are described, for example, in WO 89/06644 and WO 94/11337.

US Patent No. 7008637 describes the importance of the elimination of peak serum levels of tolterodine and its active metabolite through controlled release of tolterodine for an extended period of time, such as through a once-daily administration form, while maintaining the desired effect on the bladder, indeed gives a significant reduction of the (already low) side-effects, particularly dry mouth, compared with those obtained for the same total dosage of immediate release tablets over the same period. In other words, eliminating the peak serum levels of the active moiety affects the adverse effects, and particularly dry mouth, more than the desired effect on the detrusor activity, simultaneously as the flattening of the serum concentration does not lead to loss of activity or increased incidence of urinary retention or other safety concerns. Thus, in addition to the convenience advantage of controlled release administration, one may either (i) for a given total dosage of tolterodine, reduce the side-effects, such as dry mouth, or (ii) for a given level of acceptable side-effects, increase the dosage of tolterodine to obtain an increased effect on the bladder, if desired. However, this patent teaches a transdermal route of administration, which is clearly less desirable.

### SUMMARY OF THE INVENTION

The background art does not teach or suggest a pharmaceutical preparation for a BCS Class I drug wherein the core comprises the drug and a cellulosic polymer, and a coating layered on the core comprises an extended release polymer which is not an enteric coating polymer.

There is thus a widely recognized need for, and it would be highly advantageous to have, an extended release coated tablet featuring a BCS Class I drug, such as metoprolol or a salt thereof, for example, metoprolol succinate or metoprolol tartrate, or tolterodine or a salt thereof such as tolterodine tartrate.

The present invention overcomes these drawbacks of the background art by providing a coated tablet formulation for a BCS Class I drug, such as metoprolol salts (including metoprolol succinate and metoprolol tartrate), or tolterodine or a salt thereof (such as tolterodine tartrate), as well as methods of preparation and administration thereof.

According to some embodiments, there is provided a composition for a Biopharmaceuticals Classification System (BCS) Class I drug, comprising a core comprising the BCS Class I drug and a cellulosic polymer, and a coating comprising an extended release polymer, with the proviso that said extended release polymer is not an enteric coating polymer

In some embodiments, the BCS Class I drug comprises metoprolol or a salt thereof, preferably metoprolol succinate.

In some embodiments, the BCS Class I drug comprises tolterodine.

The tablet core optionally further comprises a polyacrylate polymer, such as, for example Eudragit®30.

According to some embodiments, the tablet core optionally and preferably further comprises one or more of a binder, a glidant, a lubricant, a disintegrant, or a filler, or a combination thereof.

The filler optionally and preferably comprises microcrystalline cellulose. Microcrystalline cellulose optionally comprises one or both of microcrystalline cellulose PH 101 or microcrystalline cellulose PH 102.

The binder is optionally and preferably at least one of Povidone (PVP: polyvinyl pyrrolidone) such as for example and without limitation Povidone K-30, low molecular weight HPC (hydroxypropyl cellulose), low molecular weight HPMC (hydroxypropyl methylcellulose) such as HPMC 2208 or 2910 for example, carboxymethyl cellulose, hydroxylethyl cellulose, ethylcellulose, gelatin polyethylene oxide, acacia, dextrin, magnesium aluminum silicate and starch. Optionally and preferably a combination of binders is used, for example and without limitation, PVP and HPMC in combination. Microcrystalline cellulose is also used as a dry binder in the formulation as an extragranular excipient. For this purpose, microcrystalline cellulose optionally comprises one or both of microcrystalline cellulose PH 102 or microcrystalline cellulose PH 200.

The lubricant optionally and preferably comprises magnesium stearate.

The glidant optionally and preferably comprises talc or silica colloidal anhydrous, or a combination thereof.

According to at least some embodiments of the present invention, the tablets are prepared by wet granulation, preferably with an aqueous solvent such as ethanol for example; compression of the wet granulation into tablets; and coating of the tablets.

According to at least some embodiments of the present invention, the tablet coating does not comprise an acrylic polymer.

According to further features in any of the embodiments of the invention, the coating optionally and preferably comprises any type of water insoluble polymer that is suitable for an extended release coating, including but not limited to polyvinyl acetate for example.

Optionally and preferably, the coating further comprises at least one excipient, such as, for example, a plasticizer, a glidant, a pore former, or a surfactant.

The glidant optionally and preferably comprises talc.

The pore former is optionally and preferably a hydrophilic pore former and is more preferably at least one of Povidone (PVP: polyvinyl pyrrolidone) such as (for example and without limitation) Povidone K-30; lactose monohydrate; microcrystalline cellulose; or Kollidon CL-M; or a combination thereof.

The plasticizer optionally and preferably comprises at least one or more of a citric acid ester and a phthalic acid ester. Non-limiting examples include triethyl citrate, tributyl citrate, acetyl triethyl citrate, dibutyl sebacate, diethyl phthalate, dibutyl phthalate, and triacetin. Preferably, the plasticizer comprises triethyl citrate.

The surfactant preferably comprises sodium lauryl sulfate.

Optionally, the coating may comprise a commercially available polymer dispersion, such as Kollicoat SR 30D, which features a 30% dispersion of polyvinyl acetate (27%) with PVP (2.7%) and sodium lauryl sulfate (0.3%) from BASF, although of course other such commercially available combination preparations may optionally be used, in addition to or in place of, this specific example preparation.

The coating may optionally also comprise one or more flavorings or taste masking ingredients, one or more colors and the like.

The formulation may optionally comprise a BCS Class I drug in any suitable dosage amount, including but not limited to metoprolol succinate in 23.75mg, 47.5mg, 95mg or 190 mg dosage amounts, or metoprolol tartrate in 25mg, 50mg, 100mg and 200 mg dosage amounts, for example.

There is further provided the use of metoprolol or a salt thereof according to various embodiments of the present invention for the treatment or prevention of a cardiac disease (such as, for example, one or more of hypertension, including essential hypertension; tachycardia; coronary heart disease; treatment after a myocardial infarction; treatment of heart failure; migraine prophylaxis; vasovagal syncope; treatment of long QT syndrome; and/or arrhythmias), angina attacks, hyperthyroidism, or an anxiety disease (such as one or more of performance anxiety, social anxiety disorder, or other anxiety disorders).

There is further provided the use of tolterodine or a salt thereof according to various embodiments of the present for the treatment of patients with an overactive bladder with symptoms of urinary frequency, urgency or urge incontinence. Overactive urinary bladder encompasses detrusor instability, detrusor hyperreflexia, urge incontinence, urgency and urinary frequency.

As used herein, the term "treating" includes curing a condition, treating a condition, preventing a condition, treating symptoms of a condition, curing symptoms of a condition, ameliorating symptoms of a condition, treating effects of a condition, ameliorating effects of a condition, and preventing results of a condition. As used herein the term "about" refers to ± 10 %.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 shows that the composition of Example 1 (squares) showed a highly similar dissolution profile to that of the reference product (diamonds) for the 190 mg dosage form;
FIG. 2 shows similar results for the 95 mg dosage form (same symbols as for Figure 1); and
FIGS. 3 and 4 show that even the cores alone had relatively slow dissolution rates.

### DESCRIPTION OF THE PREFERRED EMBODIMENTS

The present invention is of a coated tablet formulation for a BCS Class I drug, such as metoprolol or a salt thereof (for example, metoprolol succinate or metoprolol tartrate), or tolterodine or a salt thereof, as well as methods of preparation and administration thereof.

In some embodiments, the BCS Class I drug comprises metoprolol or a salt thereof, preferably metoprolol succinate.

In some embodiments, the BCS Class I drug comprises tolterodine or a salt thereof, such as tolterodine tartrate.

The tablets also optionally and preferably comprise one or more of a binder, a glidant, a lubricant, a disintegrant or a filler, or a combination thereof. The tablets are optionally and preferably formed by compression. The coating is preferably a water insoluble polymer that is suitable for an extended release formulation.

The principles and operation of the compositions and methods according to the present invention may be better understood with reference to the accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Percentages are given as weight per weight percentages over the weight of the coated tablet or over weight of core alone or coating alone, as will hereinafter be specifically defined.

### Tablet

The term "tablet" refers to substantially any structure which features a BCS Class I drug, such as metoprolol or a salt thereof, (such as metoprolol succinate or metoprolol tartrate for example, or tolterodine. The amount of BCS Class I drug may optionally comprise from about 5% to about 60%, preferably from about 5% to about 30%, more preferably from about 6% to about 26% weight per weight over the weight of the coated tablet.

In at least some embodiments, the tablet features a cellulosic polymer, preferably in a rate controlling amount. The cellulosic polymer preferably comprises one or more of low molecular weight HPC (hydroxypropyl cellulose), low molecular weight HPMC (hydroxypropyl methylcellulose) such as HPMC 2208 or 2910 for example, carboxymethyl cellulose, hydroxylethyl cellulose, ethylcellulose and the like.

The cellulosic polymer is preferably present in an amount of from about 5% to about 60%, preferably from about 10% to about 50%, more preferably from about 15% to about 40% and most preferably from about 15% to about 35% weight per weight over the weight of the core composition.

The tablet could optionally be prepared in a number of different ways which are known in the art. For example, the tablet could optionally be formed by granulation, for example and without limitation by wet granulation of a BCS Class I drug with one or more pharmaceutical excipients, preferably with an aqueous solvent such as ethanol for example. As another example, the tablet could optionally be formed by compressing a BCS Class I drug with one or more excipients. As another example, the tablet could be prepared by dry granulation of BCS Class I drug with one or more excipients.

The table core optionally further comprises a polyacrylate polymer, such as, for example Eudragit®30.

The tablets also optionally and preferably comprise one or more of a binder, a filler, a glidant, a lubricant or a disintegrant, or a combination thereof.

The binder is optionally and preferably at least one of Povidone (PVP: polyvinyl pyrrolidone) such as for example and without limitation Povidone K-30, gelatin polyethylene oxide, acacia, dextrin, magnesium aluminum silicate and starch, preferably in combination, for example with one of the above cellulosic polymers.

Apart from a cellulosic polymer or polymers, the binder is preferably present in an amount of from about 1% to about 50%, more preferably from about 5% to about 40% and most preferably from about 5% to about 25% weight per weight over the weight of the core composition.

The filler preferably comprises microcrystalline cellulose. Microcrystalline cellulose optionally comprises one or both of microcrystalline cellulose PH 101 or microcrystalline cellulose PH 102. The amount of filler optionally comprises from about 5% to about 60%, preferably from about 10% to about 50%, more preferably from about 15% to about 40%, and most preferably from about 25% to about 35% weight per weight over the weight of the core composition.

The glidant optionally comprises talc and/ or silica colloidal anhydrous or a combination thereof. If the glidant is silica colloidal anhydrous, it is optionally present in an amount of from about 0.1% to about 3%, preferably from about 0.1% to about 2% and more preferably from about 0.5% to about 1.5% weight per weight over the weight of the core composition. If the glidant is talc, it is optionally present in an amount of from about 3% to about 20%, preferably from about 5% to about 10%, weight per weight over the weight of the core composition.

The lubricant optionally and preferably comprises magnesium stearate. The amount of lubricant is optionally from about 0.1% to about 20%, preferably from about 0.5% to about 10% and more preferably from about 0.5% to about 5%.

Any suitable tabletting machine may optionally be used to compress the above ingredients into a tablet, which may then be coated with a polymer dispersion. According to at least some embodiments of the present invention, the tablets are prepared by wet granulation, preferably with an aqueous solvent such as ethanol for example; compression of the wet granulation into tablets; and coating of the tablets.

The formulation may optionally comprise a BCS Class I drug in any suitable dosage amount, including but not limited to metoprolol succinate in 23.75mg, 47.5mg, 95mg or 190 mg dosage amounts for example.

### Coating

The formulation according to the present invention optionally and preferably features a coating comprising an extended release polymer. The extended release polymer preferably comprises any type of suitable polymer or polymer combination, with the proviso that the polymer or polymer combination does not comprise an enteric coating polymer.

According to further features in any of the embodiments of the invention, the coating optionally and preferably comprises any type of water insoluble polymer, that is suitable for an extended release coating, including but not limited to polyvinyl acetate for example. The water insoluble polymer is preferably present in an amount of from about 20% to about 80%, more preferably from about 35% to about 65% and most preferably 50% to about 60% weight per weight over the weight of the coating composition.

Optionally and preferably, the coating further comprises at least one excipient, such as, for example, a glidant, a pore former, a surfactant, a lubricant, or a plasticizer.

The glidant optionally comprises talc or titanium dioxide; preferably present in an amount of from 10% to about 50%, more preferably from about 20% to about 40%, and most preferably from about 23% to about 35%, weight per weight over the weight of the coating composition.

The pore former is optionally and preferably a hydrophilic pore former and is more preferably at least one of Povidone (PVP: polyvinyl pyrrolidone) such as (for example and without limitation) Povidone K-30; lactose monohydrate; microcrystalline cellulose; or Kollidon CL-M; or a combination thereof. If the pore former comprises Povidone K-30, it is preferably present in an amount of from about 5% to about 30%, preferably from about 10% to about 20% and most preferably from about 17% to about 19%, weight per weight over the weight of the coating composition.

The plasticizer optionally and preferably comprises one or more of a citric acid ester and a phthalic acid ester. Non-limiting examples include triethyl citrate, tributyl citrate, acetyl triethyl citrate, dibutyl sebacate, diethyl phthalate, dibutyl phthalate, and triacetin. Preferably, the plasticizer comprises triethyl citrate. The plasticizer is present in an amount of optionally from about 0.01% to about 5%, preferably from about 0.05% to about 3% and more preferably from about 0.1% to about 2% weight per weight over the weight of the coating composition.

The surfactant preferably comprises sodium lauryl sulfate.

Optionally, the coating may comprise a commercially available polymer dispersion, such as Kollicoat SR 30D, which features a 30% dispersion of polyvinyl acetate (27%) with PVP (2.7%) and sodium lauryl sulfate (0.3%) from BASF, although of course other such commercially available combination preparations may optionally be used, in addition to or in place of, this specific example preparation.

The coating may optionally also comprise one or more flavorings or taste masking ingredients, one or more colors and the like.

### Preparation of formulations according to the present invention

The preparation of the compositions of the present invention is described first with reference to the following general description and then with reference to the following non-limiting examples of the preparation and application of the compositions of the present invention.

According to some embodiments, there is provided a composition for a metoprolol salt, such as metoprolol succinate and/or metoprolol tartrate, comprising a tablet comprising the metoprolol salt, and a coating for coating the tablet, wherein the coating comprises an extended release polymer, with the proviso that the extended release polymer is not an acrylic polymer. In such embodiments, an amount of metoprolol salt optionally and preferably comprises from about 5% to about 60%, more preferably from about 10% to about 50%, even more preferably from about 15% to about 40%, and most preferably from about 10% to about 30% weight per weight over the weight of the coated tablet.

According to some embodiments, there is provided a composition for a tolterodine salt, such as tolterodine tartrate, comprising a tablet comprising the tolterodine salt, and a coating for coating the tablet, wherein the coating comprises an extended release polymer, with the proviso that the extended release polymer is not an acrylic polymer.

A tablet comprising a BCS Class I drug according to any of the embodiments of the present invention could optionally be prepared in a number of different ways which are known in the art. For example, the tablet could optionally be formed by granulation, and without limitation by wet granulation of a BCS Class I drug with one or more pharmaceutical excipients, preferably with a solvent such as ethanol for example. As another example, the tablet could optionally be formed by compressing a BCS Class I drug with one or more excipients. As another example, the tablet could be prepared by dry granulation of a BCS Class I drug with one or more excipients.

According to preferred embodiments, the tablet is preferably formed by wet granulation of a BCS Class I drug with a solvent such as ethanol for example, followed by compression of the granulate material into tablets.

The coating solution is preferably prepared by dispersing the water insoluble polymer or polymer combination in water, and mixing the dispersion with one or more other ingredients, including but not limited to a glidant, a pore former, a surfactant, a lubricant, and a plasticizer.

The coating solution is then layered over the tablet to form the composition of the present invention.

The coating may optionally be applied to the tablets by conventional coating techniques such as, for instance, perforated pan coating, pan coating, fluidized bed coating, fluidized bed bottom sprayed coating or a Turbo Jet-Technology for the production of large amounts. Coating may be performed using a Fluidized Bed Processor (such as that of Glatt Gmbh). A fluidized bed is a bed of solid particles which are suspended in a stream of air or gas passing upward through the particles, in which the coating material is aerosolized. As the air travels through the particle bed, the particles are mixed in the stream of gas or air with the coating material, and so are coated but are also dried. Alternatively a turbo coating system may optionally be used.
In perforated pans, airflow through the pan (and the product being coated) is facilitated by the fully perforated cylindrical portion of the pan. The structure of the drum enables air to be introduced into the interior of the pan, drawn through the product being coated, and ultimately vented.

Compositions of the present invention may, if desired, be presented in a pack or dispenser device, such as an FDA approved kit, which may contain one or more dosage forms containing the active ingredient. The pack may, for example, comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above description, illustrate the invention in a non limiting fashion.

### Example 1

For this Example, two different dosage forms were prepared; one dosage form containing 190 mg of metoprolol succinate (referred to herein as the "190mg" form) and one dosage form containing 95 mg

| **Core:** | **Mg/tab** | **Mg/tab** |
|---|---|---|
| Metoprolol succinate (API) | 190 | 95 |
| Microcrystalline cellulose PH 101 | 198.5 | 99.25 |
| HPMC 2208 (Methocel K-100M) | 112 | 56 |
| Povidone K-30 | 56 | 28 |
| Microcrystalline cellulose PH 102 | 70 | 35 |
| Talc | 35 | 17.5 |
| Silica colloidal anhydrous | 3.5 | 1.75 |
| Mg stearate | 35 | 17.5 |
| **Total core** | **700** | **350** |
| **Coating:** | | |
| Kollicoat SR 30D (Polyvinyl acetate 30% dispersion) | 15.5 | 20 |
| Povidone K-30 | 5.425 | 5 |
| Triethyl citrate | 0.775 | 1 |
| Talc | 6.300 | 9 |
| **Total weight (core + coating)** | **728** | **386** |

Metoprolol succinate, microcrystalline cellulose PH 101, HPMC 2208 (Methocel K-100M) and part of Povidone K-30 were mixed together to form a blend.

Part of Povidone K-30 was dissolved in ethanol, to form a binder solution which was further sprayed on the blend. After granulation and drying process at temperature of 65C, the granulate was sieved and microcrystalline cellulose PH 102 was added. Talc, silica colloidal anhydrous and magnesium stearate were sieved and added to form the final blend.

### Compression

The above mixture was compressed to form a tablet. The core units were compressed to tablets using a tabletting machine. The hardness ranged between 30 kilo Newton (kN) and 150 kN for this illustrative, non-limiting example, although of course the tablets are not limited to this range.

### Coating

The coating was produced and was then applied over the compressed tablet. The coating process was performed in a drum coater using X spray nozzle.

### Example 2

For this Example, two different dosage forms were prepared; one dosage form containing 47.5 mg of metoprolol succinate (referred to herein as the "47.5mg" form) and one dosage form containing 23.75 mg of metoprolol succinate (referred to herein as the "23.75mg" form). The dosage forms differ in a number of respects beyond the different amounts of the active ingredient.

| **Core:** | **Mg/tab (high dose metoprolol)** | **Mg/tab (low dose metoprolol)** |
|---|---|---|
| Metoprolol succinate (API) | 47.5 | 23.75 |
| Microcrystalline cellulose PH 102 | 113.25 | 137 |
| HPMC 2208 (Methocel K-100M) | 100 | 100 |
| Povidone K-30 | 28 | 28 |
| Microcrystalline cellulose PH 200 | 35 | 35 |
| Talc | 17.5 | 17.5 |
| Silica colloidal anhydrous | 1.75 | 1.75 |
| Mg stearate | 7 | 7 |
| **Total core** | **350** | **350** |
| **Coating:** | | |
| Kollicoat SR 30D (Polyvinyl acetate 30% dispersion) | 16.6 | 18.5 |
| Povidone K-30 | 5.81 | 6.475 |
| Triethyl citrate | 0.83 | 0.925 |
| Talc | 6.76 | 7.6 |
| **Total coating** | 30 | 33.5 |
| **Total weight (core + coating)** | 380 | 383.5 |

The compositions were prepared as for Example 1 above, with microcrystalline cellulose (MCC) PH 102 instead of MCC PH 101 mixed in the granulate, and MCC PH 200 instead of MCC PH 102 added after granulation and drying.

### Example 3

| **Core** | **Concentration (w/w** |
|---|---|
| Metoprolol succinate (API) | 20-35% |
| Microcrystalline cellulose PH 101 | 15%-30% |
| HPMC 2208 (Methocel K-100M) | 15%-30% |
| Polyacrylate dispersion 30% (Eudragit NE 30D)* | 5%-20% |
| Talc | 1%-10% |
| Silica collidal anhydrous | 0.1%-2% |
| Sodium stearyl fumarate | 1%-5% |

| **Coating** | **Concentration (w/w)** |
|---|---|
| Kollicoat SR 30D (solid content) | 50%-70% |
| Triethyl citrate | 0.5%-5% |
| Povidone K-30 | 10%-30% |
| Talc | 1%-5% |
| Purified water | Not present in the final tablet |
| | |

| | |
|---|---|
| * Percent weight/weight concentration given as the amount of dry solids | |

The compositions were prepared as for Example 1 above, using Eudragit N E 30D instead of Povidone K-30, and sodium stearyl fumarate instead of magnesium stearate.

### Example 4:

| **Core** | **Concentration (w/w)** |
|---|---|
| Tolterodine tartrate | 2-5% |
| HPMC 2208 (Methocel K-100M) | 24-45% |
| Microcrystalline cellulose PH 101 | 25-45% |
| Microcrystalline cellulose PH 102 | 15-35% |
| Povidone (K-30) | 2-10% |
| Silica colloidal anhydrous | 0-1% |
| Magnesium stearate | 0-1% |

| **Coating** | **% (w/w)** |
|---|---|
| Kollicoat SR 30D (solid content) | 50%-70% |
| Triethyl citrate | 0.5%-5% |
| Povidone K-30 | 10%-30% |
| Talc | 1%-5% |
| Purified water | Not present in the final tablet |

The compositions were prepared as for Example 1 above, with tolterodine tartrate replacing metoprolol succinate.

### Example 5: In vitro dissolution study

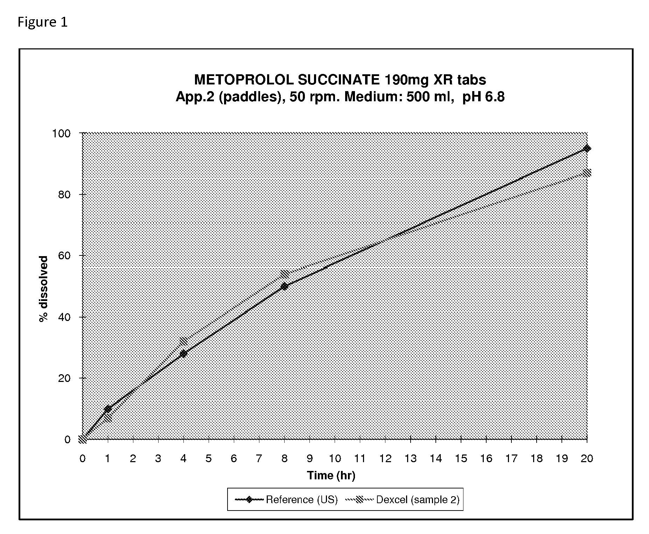

A comparative dissolution study of the composition of Example 1, 190 mg dosage form, was performed in comparison to a metoprolol succinate reference product which contains a regular dosage of metoprolol succinate (in this Example, as reference, Toprol® XL extended release tablets, in a 200mg dosage form were used) in phosphate buffer pH 6.8, using apparatus II (paddle) at 50 rpm. Figure 1 shows that surprisingly, the composition of Example 1 (squares) showed a highly similar dissolution profile to that of the reference product (diamonds), even though the reference product is a tablet of compressed pellets, further coated with a non-functional, more 'cosmetic' coating.

Table 1 below shows the above data, again demonstrating the highly similar dissolution values for both compositions.

**Table 1 - in vitro dissolution**

| **TIME(h)** | **Composition of Example 1** | **Reference composition** |
|---|---|---|
| 0 | 0 | 0 |
| 1 | 7 | 10 |
| 4 | 32 | 28 |
| 8 | 54 | 50 |
| 20 | 87 | 95 |

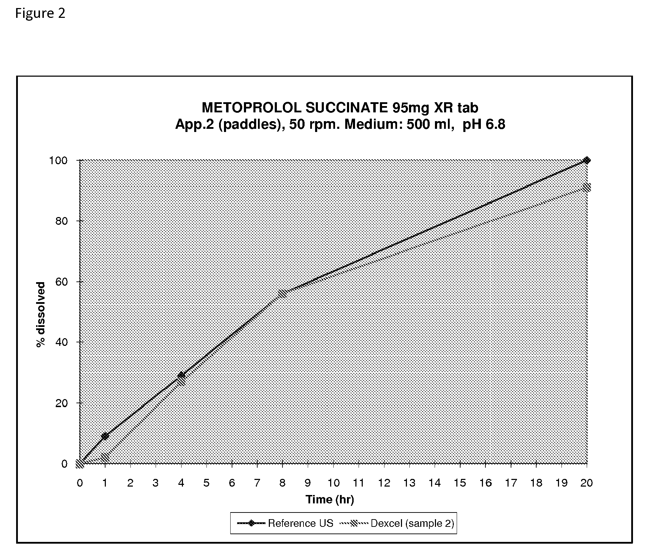

Figure 2 shows similar results for the 95 mg dosage form (same symbols as for Figure 1).

Table 2 below shows the above data, again demonstrating the highly similar dissolution values for both compositions.

**Table 2 - in vitro dissolution**

| **TIME(h)** | **Composition of Example 1** | **Reference composition** |
|---|---|---|
| 0 | 0 | 0 |
| 1 | 2 | 9 |
| 4 | 27 | 29 |
| 8 | 56 | 56 |
| 20 | 91 | 100 |

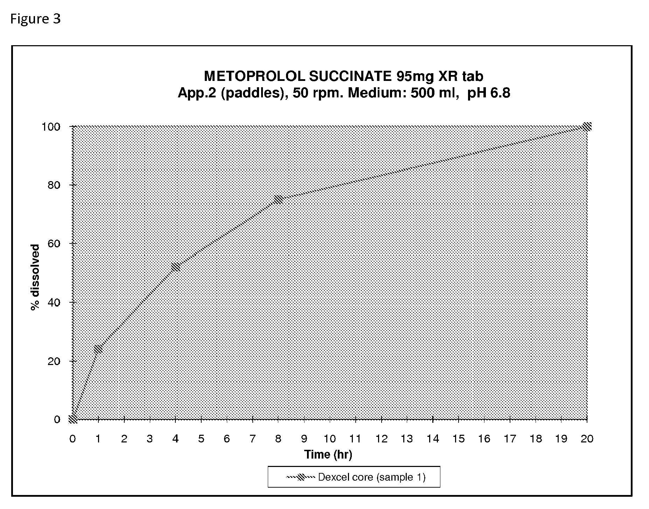

The uncoated cores were also tested for their in vitro dissolution rates according to the above test, for the 95 mg dosage amount of Example 1. Figure 3 shows that even the cores alone had relatively slow dissolution rates; complete dissolution occurred in hours. Table 3 below also shows this data.

**Table 3 - in vitro dissolution of cores alone**

| **TIME(h)** | **Cores alone of Example 1** |
|---|---|
| 0 | 0 |
| 1 | 24 |
| 4 | 52 |
| 8 | 75 |
| 20 | 100 |

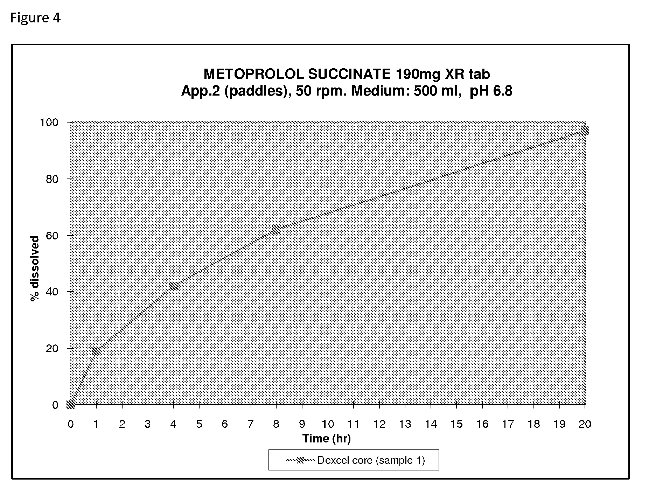

In addition, the uncoated cores were also tested for their in vitro dissolution rates according to the above test, for the 190 mg dosage amount of Example 1. Figure 4 shows that even the cores alone had relatively slow dissolution rates; complete dissolution occurred in hours. Table 4 below also shows this data.

**Table 4 - in vitro dissolution of cores alone**

| **TIME(h)** | **Cores alone of Example 1** |
|---|---|
| 0 | 0 |
| 1 | 19 |
| 4 | 42 |
| 8 | 62 |
| 20 | 97 |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A composition for a Biopharmaceutics Classification System (BCS) Class I drug, comprising a core comprising the highly soluble active pharmaceutical ingredient, the core comprising a cellulosic polymer, and a coating for coating said core, wherein said coating comprises an extended release polymer, with the proviso that said extended release polymer is not an enteric coating polymer.

2. The composition of claim 1, wherein said Biopharmaceutics Classification System (BCS) Class I drug is selected from the group consisting of metoprolol salt, tolterodine, and salts thereof.

3. The composition of claim 1, wherein said extended release polymer comprises a water insoluble polymer, optionally in an amount of from about 20% to about 80% weight per weight over the weight of the coating composition.

4. The composition of claim 3, wherein said water insoluble polymer comprises polyvinyl acetate.

5. The composition of claim 1, wherein said core further comprises a polyacrylate polymer, preferably Eudragit®30.

6. The composition of claim 1, said core further comprising one or more of a binder, a glidant, a lubricant, a disintegrant, or a filler, or a combination thereof,
wherein said filler in said core is optionally present in an amount of from about 5% to about 60% weight per weight over the weight of the core composition,
wherein said binder in said core is optionally present in an amount of from about 1% to about 50% weight per weight over the weight of the core composition,
wherein said lubricant in said core is optionally present in an amount of from about 0.1% to about 20% weight per weight over the weight of the core composition,
wherein said glidant in said core is optionally present in an amount of from about 0.1% to about 20% weight per weight over the weight of the core composition.

7. The composition of claim 6, wherein said filler comprises microcrystalline cellulose, optionally comprising at least one of microcrystalline cellulose PH 101 and microcrystalline cellulose PH 102,
wherein said binder optionally comprises one or more of polyvinyl pyrrolidone (PVP), low molecular weight HPC (hydroxypropyl cellulose), low molecular weight HPMC (hydroxypropyl methylcellulose), carboxymethyl cellulose, hydroxylethyl cellulose, ethylcellulose, gelatin polyethylene oxide, acacia, dextrin, magnesium aluminum silicate and starch, or combinations thereof,
wherein said lubricant optionally comprises magnesium stearate,
wherein said glidant in said core optionally comprises talc or silica colloidal anhydrous, or a combination thereof.

8. The composition of claim 1, wherein said cellulosic polymer is present in a sufficient quantity to be rate controlling, optionally in an amount of from about 5% to about 60% weight per weight over the weight of the core composition.

9. The composition of claim 7, wherein said binder comprises PVP and HPMC, wherein said PVP optionally comprises Povidone K-30 and wherein said HPMC optionally comprises one or more of HPMC 2208 or 2910.

10. The composition of any of the above claims, wherein said coating further comprises one or more of a plasticizer, a glidant, a pore former, or a surfactant,
wherein said glidant optionally comprises talc or titanium dioxide, wherein said talc is optionally present in an amount of from about 10% to about 50% weight per weight over the weight of the coating,
wherein said pore former optionally comprises a hydrophilic pore former and is more preferably at least one of Povidone (PVP: polyvinyl pyrrolidone); lactose monohydrate; microcrystalline cellulose; or Kollidon CL-M; or a combination thereof, wherein said Povidone optionally comprises Povidone K-30, optionally in an amount of from about 5% to 30% weight per weight over the weight of the coating,
wherein said plasticizer in said coating is optionally present in an amount of from about 0.01% to about 5% weight per weight over the weight of the coating,
wherein said plasticizer optionally comprises at least one or more of a citric acid ester and a phthalic acid ester,
wherein said surfactant optionally comprises sodium lauryl sulfate.

11. The composition of any of the above claims, wherein said core comprises a tablet.

12. A composition for a metoprolol salt, comprising a tablet comprising a metoprolol salt, and a coating for coating said tablet, wherein said coating comprises an extended release polymer, with the proviso that said extended release polymer is not an acrylic polymer.

13. The composition of claim 2, wherein said metoprolol salt comprises one or more of metoprolol succinate and metoprolol tartrate, wherein an amount of said metoprolol salt optionally comprises from about 5% to about 60% weight per weight over the weight of the coated tablet.

14. A method for producing the composition of claim 12, comprising preparing the tablet; compression of the wet granulation into tablets; and coating of the tablets.
